# EUROPEAN PATENT APPLICATION

(11) **EP 4 600 369 A1**
(43) Date of publication of application: **13.08.2025**
(21) Application number: 24781281.1
(22) Date of filing: 29.03.2024
(51) Int. Cl.: C12P 13/06, C12N 1/20, C12N 1/14, C12R 1/72, C12R 1/125, C12R 1/15

(54) **PROCESS FOR PRODUCING O-ACETYL HOMOSERINE USING MICROBIAL FERMENTATION**

(30) Priority: 31.03.2023 KR 20230043099
(71) Applicant: CJ CheilJedang Corporation, Seoul 04560 (KR)
(72) Inventor: KIM, Jung Ho, Seoul 04560 (KR); HONG, Hyeongpyo, Seoul 04560 (KR); PARK, Sang Min, Seoul 04560 (KR)
(74) Representative: Meissner Bolte Partnerschaft mbB
(86) International application number: PCT/KR2024/004011
(87) International publication number: WO 2024/205291

(57) **Abstract**

The present disclosure relates to a method for preparing O-acetyl-homoserine, which comprises a main culture step of culturing an O-acetyl-homoserine producing strain in a medium to produce O-acetyl-homoserine, and in which a potassium salt is supplied to the medium. According to the present disclosure, O-acetyl-homoserine productivity (g/L/hr) is improved by additionally supplying potassium ions in the form of a potassium salt to the main medium in the culture method for producing O-acetyl-homoserine.

## Description

### [Technical Field]

The present disclosure relates to a process technology for preparing O-acetyl-homoserine utilizing microbial fermentation.

### [Background Art]

According to the prior art, in the production of O-acetyl-homoserine products using a *Corynebacterium* strain with improved O-acetyl-homoserine producing ability, there is a problem in that the production volume is limited by the use of a fermenter with limited resources to produce a fermented medium comprising O-acetyl-homoserine even though a strain with improved producing ability is used.

### [Citation List]

### [Patent Document]

Patent Document 1: US 2017-0101655 A1

### [Disclosure]

### [Technical Problem]

An object of the present disclosure is to improve productivity by adding a potassium salt during the O-acetyl-homoserine preparing process utilizing microbial fermentation.

### [Technical Solution]

This will be specifically described as follows. Each description and each embodiment disclosed in the present disclosure may also be applied to other descriptions and other embodiments, respectively. That is, all combinations of various elements disclosed in the present disclosure fall within the scope of the present disclosure. Further, the scope of the present disclosure is not limited by the specific description below.

The present disclosure relates to an O-acetyl-homoserine preparing process utilizing microbial fermentation, and is a technology for improving O-acetyl-homoserine producing ability by adding potassium hydroxide to a medium during the fermentation process for preparing O-acetyl-homoserine.

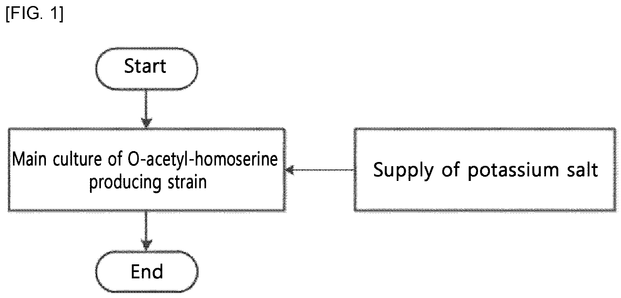

FIG. 1 is a flow chart illustrating a method for preparing O-acetyl-homoserine according to the present disclosure.

Referring to FIG. 1, the method for preparing O-acetyl-homoserine according to the present disclosure comprises a main culture step of culturing an O-acetyl-homoserine producing strain in a medium to produce O-acetyl-homoserine, in which a potassium salt is supplied to the medium. Hereinafter, each step will be described in detail.

The main culture step is a step of preparing a fermented medium comprising O-acetyl-homoserine using an O-acetyl-homoserine producing strain.

The term "fermented product" in the present disclosure may mean the result of enzymatic or metabolic decomposition of an organic substance using a microorganism. For example, the fermented product may comprise a culture itself obtained by culturing a microorganism in a culture medium, or a concentrate, dried product, or lyophilisate of a culture obtained by removing the strain from this culture. Additionally, in this case, the fermented medium may comprise the entire fermented product including an amino acid or may be a fermented product comprising an amino acid from which impurities have been removed.

The "O-acetyl-homoserine producing strain" used in the main culture step comprises both wild type microorganisms and microorganisms that have undergone natural or artificial genetic modification, is a microorganism of which a specific mechanism is weakened or strengthened by causes such as insertion of external genes or enhancement or inactivation of endogenous genes, and may be a microorganism that has genetic modification for the production of a target protein or amino acid.

The O-acetyl-homoserine producing strain of the present disclosure may be, but is not limited to, a microorganism that naturally has O-acetyl-homoserine producing ability, or a microorganism obtained by imparting O-acetyl-homoserine producing ability to a parent strain that does not have O-acetyl-homoserine producing ability. Specifically, in the present disclosure, a microorganism producing O-acetyl-homoserine or a target product, or a microorganism having the ability to produce O-acetyl-homoserine or a target product, may be a microorganism in which some of the genes in a target protein or target product biosynthesis pathway are strengthened or weakened, or some of the genes in a target protein or target product degradation pathway are strengthened or weakened. "Enhancement" of or "increase" in the O-acetyl-homoserine producing ability of the microorganism of the present disclosure means that the O-acetyl-homoserine producing ability of the microorganism of the present disclosure is improved compared to that of microorganisms other than the microorganism of the present disclosure, a parent strain, or an unmodified microorganism. For example, the microorganism of the present disclosure may have O-acetyl-homoserine producing ability that is improved by about 1% or more, 10% or more, 100% or more, 200% or more, 500% or more, 1000% or more, 1100% or more, 1200% or more, or 1300% or more compared to the O-acetyl-homoserine producing ability of other microorganisms, and may have O-acetyl-homoserine producing ability that is improved by about 1.01 times or more, 2 times or more, 5 times or more, 10 times or more, 11 times or more, 12 times or more, or 13 times or more compared to the O-acetyl-homoserine producing ability of other microorganisms, but is not limited thereto. The term "about" comprises, but is not limited to, a range comprising all of ±0.5, ±0.4, ±0.3, ±0.2, ±0.1, and the like, and all numerical values in a range equal to or similar to the numerical value following the term "about."

The above-described microorganism used in the main culture step may be at least one selected from the group consisting of the yeast *Candida famata,* the ascomycetes *Eremothecium ashbyii* and *Ashbya gossypii,* and the bacteria *Bacillus subtilis* and *Corynebacterium sp.*

In a case where the microorganism used in the main culture step is a microorganism belonging to the genus *Corynebacterium,* the microorganism may specifically be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris, Corynebacterium crenatum,* or *Corynebacterium flavescens,* and may more specifically be *Corynebacterium glutamicum,* but is not limited thereto.

Microorganisms belonging to the genus *Corynebacterium,* particularly *Corynebacterium glutamicum,* are gram-positive microorganisms that are widely used in the production of L-amino acids and other useful substances. In order to produce L-amino acids and other useful substances, various studies are being conducted to develop microorganisms and fermentation process technologies for highly efficient production. For example, target substance-specific approaches are mainly used, such as increasing the expression of genes encoding enzymes involved in the biosynthesis of L-tryptophan, L-valine, L-isoleucine, L-leucine, L-histidine, O-acetyl-homoserine or L-threonine or removing genes unnecessary for the biosynthesis. In the present disclosure as well, a fermented medium comprising an amino acid can be prepared using a strain belonging to the genus *Corynebacterium.*

According to an embodiment of the present disclosure, the O-acetyl-homoserine producing strain may be a microorganism of the genus *Escherichia,* which exhibits weakened or inactivated endogenous citrate synthase protein activity and has O-acetyl-homoserine producing ability enhanced compared to microorganisms of which the citrate synthase protein activity is not yet weakened or inactivated, among microorganisms of the genus *Escherichia* that produce O-acetyl-homoserine. In particular, KCCM11146P-A167 (deposited on November 22, 2013, at the KOREAN CULTURE CENTER FOR MICROORGANISMS (KCCM), an international depository under the Budapest Treaty, and assigned the deposit number KCCM11483P), a representative strain with weakened citrate synthase activity that has been verified to have excellent O-acetyl-homoserine producing ability, can be used as the O-acetyl-homoserine producing strain of the present disclosure. In a case of using the above-described microorganism having O-acetyl-homoserine producing ability, O-acetyl-homoserine can be produced with a higher yield in a more environmentally friendly manner compared to chemical synthesis. The produced O-acetyl-homoserine can be used as a precursor for methionine and acetic acid synthesis by O-acetyl-homoserine sulfhydrylase, the precursor can be bioconverted into L-methionine at a high efficiency, and the converted L-methionine can be widely used in the production of animal feed or animal feed additives, as well as human food or food additives.

In the main culture step, the culture of an O-acetyl-homoserine producing strain can be carried out under appropriate culture conditions known in the art. Such a culture process can be easily adjusted and used by those skilled in the art depending on the selected strain. Specifically, the culture may be batch, continuous, and fed-batch culture, but is not limited thereto.

As disclosed in FIG. 1, a potassium salt is supplied during the main culture step. In the main culture step, potassium salt is added to regulate the potassium (K⁺) concentration in the medium. In some cases, it is possible to adjust only the potassium concentration without affecting the phosphorus (P) concentration in a case of adjusting the potassium concentration in the medium by adding a potassium salt in a form other than potassium phosphate. Potassium is known to be essential for enzyme activity involved in protein synthesis during microbial growth, and the efficiency of O-acetyl-homoserine production by an O-acetyl-homoserine producing strain can be improved by adjusting the potassium concentration through the addition of KOH. Because potassium is involved in the membrane potential of microorganisms, the potential difference can be maintained if the potassium concentration is maintained in a certain range, and the maintenance of the potential difference affects the osmotic pressure of microorganisms and ultimately the efficiency of O-acetyl-homoserine preparation.

The potassium salt utilized in the main culture step may be at least one selected from the group consisting of potassium hydroxide (KOH), potassium acetate (KAc), potassium chloride (KCI), potassium sulfate (K₂SO₄), and potassium carbonate (K₂CO₃).

A potassium salt may be added so that the potassium ion concentration in the medium is 0.3 g/L to 1.5 g/L. At this time, the concentration of potassium salt added can be determined by considering the change in O-acetyl homoserine producing efficiency depending on the potassium ion concentration. It has been found that when the potassium ion concentration in the medium is 0.3 g/L to 1.5 g/L, the effect of increasing enzyme activity and maintaining the membrane potential difference achieved by the addition of potassium is excellent, and the increase in O-acetyl-homoserine producing efficiency due to the increase in potassium concentration is the highest. The potassium ion concentration may be, in some cases, 0.3 g/L to 1.0 g/L, 0.3 g/L to 0.5 g/L, 0.5 g/L to 1.5 g/L, 0.5 g/L to 1.0 g/L, or 1.0 g/L to 1.5 g/L.

When KOH is used as a potassium salt to achieve the potassium salt concentration level described above, KOH may be added so that the concentration in the medium is 0.4 g/L to 2.2 g/L. In some cases, KOH may be added so that the concentration in the medium is 0.4 g/L to 1.5 g/L, 0.4 g/L to 1.0 g/L, 1.0 g/L to 2.2 g/L, 1.0 g/L to 1.5 g/L, or 1.5 g/L to 2.2 g/L.

In a case where potassium acetate (KAc) is used as the potassium salt, potassium acetate may be added so that the KAc concentration in the medium is 2.6 g/L to 3.9 g/L. In some cases, potassium acetate may be added so that the concentration in the medium is 2.6 g/L to 3.5 g/L, 2.6 g/L to 3.0 g/L, 3.0 g/L to 3.9 g/L, 3.0 g/L to 3.5 g/L, or 3.5 g/L to 3.9 g/L.

In a case where potassium chloride (KCI) is used as the potassium salt, potassium chloride may be added so that the KCI concentration in the medium is 2.0 g/L to 3.0 g/L. In some cases, potassium chloride may be added so that the KCI concentration in the medium is 2.0 g/L to 2.5 g/L or 2.5 g/L to 3.0 g/L.

In a case where potassium sulfate (K₂SO₄) is used as the potassium salt, potassium sulfate may be added so that the K₂SO₄ concentration in the medium is 4.6 g/L to 6.9 g/L. In some cases, potassium sulfate may be added so that the K₂SO₄ concentration in the medium is 4.6 g/L to 6.5 g/L, 4.6 g/L to 6.0 g/L, 4.6 g/L to 5.5 g/L, 4.6 g/L to 5.0 g/L, 5.0 g/L to 6.9 g/L, 5.0 g/L to 6.5 g/L, 5.0 g/L to 6.0 g/L, 5.0 g/L to 5.5 g/L, 5.5 g/L to 6.9 g/L, 5.5 g/L to 6.5 g/L, 5.5 g/L to 6.0 g/L, 6.0 g/L to 6.9 g/L, 6.0 g/L to 6.5 g/L, or 6.5 g/L to 6.9 g/L.

In a case where potassium carbonate (K₂CO₃) is used as the potassium salt, potassium carbonate may be added so that the K₂CO₃ concentration in the medium is 3.7 g/L to 5.5 g/L. In some cases, potassium carbonate may be added so that the K₂CO₃ concentration in the medium is 3.7 g/L to 5.0 g/L, 3.7 g/L to 4.5 g/L, 3.7 g/L to 4.0 g/L, 4.0 g/L to 5.5 g/L, 4.0 g/L to 5.0 g/L, 4.0 g/L to 4.5 g/L, 4.5 g/L to 5.5 g/L, 4.5 g/L to 5.0 g/L or 5.0 g/L to 5.5 g/L.

The potassium salt may be added before the start of the main culture or during the main culture. In other words, the potassium salt may be added to the medium in advance before the start of the main culture, or it may be added to the medium at an appropriate timepoint during the main culture.

In the main culture step, foaming can be suppressed by using an antifoaming agent such as fatty acid polyglycol ester. At this time, oxygen or oxygen-containing gas and air can be continuously injected into the culture to maintain the aerobic state. The temperature of the culture can be maintained between 20°C and 45°C. The main culture may be continuously performed until the desired maximum production volume of O-acetyl-homoserine is obtained. For this purpose, the main culture may be performed for 10 to 70 hours. O-acetyl-homoserine may be released into the culture medium or contained within the cells.

After the main culture step is performed, a step of separating O-acetyl-homoserine from the fermented medium and a step of commercializing the separated O-acetyl-homoserine may additionally be performed.

For example, the fermented medium prepared after the main culture step is performed may be concentrated, and O-acetyl-homoserine wet crystals produced during the concentration process may be separated. In this process, water in the fermented medium is removed to precipitate O-acetyl-homoserine in the form of crystals. The concentration may be performed by various methods. The concentration may be performed in a conventional concentrator (for example, a paddle dryer, slurry drying equipment, a vacuum concentrator, a forced circulation concentrator, a thin film concentrator, or a rotary concentrator) at the discretion of those skilled in the art.

In order to separate the O-acetyl-homoserine wet crystals precipitated through concentration from the mother liquor, a solid-liquid separator such as a pressure-reducing membrane filtration device, a pressure-increasing membrane filtration device, or a centrifugal separator may be used, but the separation is not limited thereto.

The mother liquor remaining after the O-acetyl-homoserine wet crystal separation may be reused for concentration. Accordingly, O-acetyl-homoserine that fails to form a wet crystal or O-acetyl-homoserine that is precipitated as a crystal having a size smaller than a certain size and remains in the mother liquor without being separated in the O-acetyl-homoserine wet crystal separating step may be reused. After the mother liquor circulation, a process of heating the fermented medium and the like may be additionally performed to re-dissolve the O-acetyl-homoserine precipitated in the form of fine crystals in the fermented medium, if necessary.

Next, a step of mixing the separated O-acetyl-homoserine wet crystals with the seed to prepare O-acetyl-homoserine mixed granules may be performed. The seed used in the step is also called a seed crystal, and may refer to a substance used as a catalyst for crystallization or granulation of a liquid. Specifically, the seed in the present disclosure may be, but is not limited to, an amino acid crystal, for example, a crystal of O-acetyl-homoserine identical to O-acetyl-homoserine comprised in the fermentation concentrate to be granulated. When the seed comes into contact with the fermented medium, the solid components present in the fermented medium bind with the seed to cause aggregation, and thus granules can be formed. The seed used in the step may have an average particle size of 150 µm to 300 µm. Specifically, seeds having an average particle size of 150 µm to 250 µm, 200 µm to 300 µm, or 200 µm to 250 µm may be used, but the seed is not limited thereto. The particle size of the seed used may ultimately affect the productivity in the preparation of granules according to the present disclosure, and may be appropriately selected by those skilled in the art by considering the desired water content and the like.

In the step, O-acetyl-homoserine mixed granules may be prepared using a mixing granulator. The mixing granulator may be to obtain granules by feeding seeds into the mixing granulator through a feeder at a constant rate while simultaneously supplying the previously obtained amino acid wet crystals. Here, "granules" are macroscopic particles, which are permanent aggregates with a larger size formed by the gathering of smaller particles such as powder, and may be particles having an average particle diameter of 50 µm to 5 mm, 75 µm to 4 mm, or 100 µm to 3 mm.

Next, the O-acetyl-homoserine mixed granules may be dried to obtain an amino acid product. In the drying step, the previously obtained O-acetyl-homoserine mixed granules are dried, and there is no limitation on the drying method. After drying, the O-acetyl-homoserine product may be obtained. The term "O-acetyl-homoserine product" in the present disclosure means a product prepared in various formulations using an amino acid substance comprised in a fermented medium. For example, an O-acetyl-homoserine product may mean a mixture comprising an amino acid in the form of granules. However, if necessary, the formulation of the O-acetyl-homoserine product may be changed without changing the inventor's spirit of the present disclosure. As described above, subsequent processes may be further performed to implement various formulations of the O-acetyl-homoserine product. The above-described O-acetyl-homoserine product may be used as an additive for animal feed, and the like, and the application thereof is not limited.

Meanwhile, prior to the main culture step, a step of culturing the O-acetyl-homoserine producing strain in a flask and subsequently a seed culture step of culturing the O-acetyl-homoserine producing strain may be performed. Flask culture and seed culture are processes of sufficiently proliferating microorganisms in a seed medium to increase the number of microorganisms required for fermentation, and these processes may be performed by common methods used in the art. By performing the steps described above, an appropriate number of O-acetyl-homoserine producing strains can be directly used in the main culture, and thus the proliferation process for increasing the number of O-acetyl-homoserine producing strains may be omitted. Hence, the efficiency of the overall O-acetyl-homoserine producing process can be improved.

According to an embodiment of the present disclosure, a medium for preparing O-acetyl-homoserine through microbial fermentation is provided.

The medium may be a composition that affords an appropriate environment for the metabolism of an O-acetyl-homoserine producing strain for O-acetyl-homoserine production. The "medium" refers to a material in which nutrients necessary for culturing microorganisms are mixed as main ingredients, and it supplies nutrients and growth factors, including water, which are essential for survival and development.

The medium of the present disclosure comprises a carbon source, a nitrogen source, a phosphorus source and a potassium salt.

The carbon source may include carbohydrates such as glucose, fructose, sucrose, maltose, mannitol, and sorbitol; alcohols such as sugar alcohols and glycerol; organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and pasteurized pretreated molasses (that is, molasses converted into reducing sugar) may be used. Other carbon sources may be variously used in proper amounts without limitation. These carbon sources may be used singly or in combination of two or more kinds thereof, but the carbon source is not limited thereto.

As the nitrogen source that can be used, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptones, NZ-amines, meat extract, yeast extract, malt extract, corn steep liquor, casein hydrolysate, fish or their decomposition products, and defatted soybean cake or its decomposition products may be used. These nitrogen sources may be used singly or in combination of two or more kinds thereof, but the nitrogen source is not limited thereto.

The phosphorus source may include potassium phosphate monobasic and potassium phosphate dibasic or sodium phosphate monobasic and sodium phosphate dibasic. As inorganic compounds, sodium chloride, calcium chloride, iron chloride, magnesium sulfate, iron sulfate, manganese sulfate, calcium carbonate and the like may be used, and the inorganic compounds may also include amino acids, vitamins and/or suitable precursors in addition to these. These components or precursors may be added to the medium in a batch or continuous manner, but the addition is not limited thereto.

As described above, the concentration of potassium salt in the medium may vary depending on the type thereof. The technical significance of this is as described above.

The medium may also comprise essential growth substances such as vitamins and metal salts, such as magnesium sulfate or iron sulfate, which are necessary for microbial growth.

The raw materials may be added to the culture in a batch or continuous manner by an appropriate method during the culture process. Hence, in the present disclosure, the term "medium" may mean not only the composition added at the start of the main culture, but also the totality of substances added during the culture process of the O-acetyl-homoserine producing strain.

### [Advantageous Effects]

According to the present disclosure, O-acetyl-homoserine productivity (g/L/hr) is improved by additionally supplying potassium ions in the form of a potassium salt to the main medium in the culture method for producing O-acetyl-homoserine.

### [Brief Description of Drawings]

FIG. 1 is a flow chart illustrating a method for preparing O-acetyl-homoserine according to the present disclosure.

### [Detailed Description of the Invention]

Hereinafter, the present disclosure will be described in more detail with reference to the Examples. However, the following Examples are merely preferred embodiments for illustrating the present disclosure, and therefore the scope of rights of the present disclosure is not intended to be limited thereto. Meanwhile, technical matters not described in this specification can be sufficiently understood and easily implemented by a person skilled in the technical field of the present disclosure or a similar technical field.

In the above, a method for preparing O-acetyl-homoserine according to an aspect of the present disclosure and the medium used in the method have been explained. Hereinafter, the advantageous effects mentioned in the present disclosure will be explained through experimental results of Examples and Comparative Examples.

### Experimental Example 1. Comparison of O-acetyl-homoserine productivity (g/L/hr) depending on concentration of potassium hydroxide (KOH) added during main culture

According to Example of the present invention, the culture was centrifuged at a low speed to remove biomass, and the obtained supernatant was separated by ion exchange chromatography and analyzed.

**[Table 1]**

| **No.** | **Concentration of KOH added (g/L)** | **K⁺ concentration added (g/L)** | **Productivity (g/L/hr)** |
|---|---|---|---|
| 1 | 0.0 | 0.0 | 6.83 |
| 2 | 0.4 | 0.3 | 7.16 |
| 3 | 0.7 | 0.5 | 7.27 |
| 4 | 1.1 | 0.8 | 7.46 |
| 5 | 1.5 | 1.0 | 7.40 |
| 6 | 2.2 | 1.5 | 7.38 |
| 7 | 2.6 | 1.8 | 6.50 |

In Experimental Example 1, culture was performed at various concentrations of KOH added during the main culture, and the culture temperature, pH, and total equivalents added, except for the concentration of KOH added, were all constant. At a concentration of KOH added of 0.4 g/L (potassium ion concentration of 0.3 g/L), the productivity increased by 4.8% compared to that when KOH was not added (potassium ion concentration of 0 g/L). At a concentration of KOH added of 0.7 g/L (potassium ion concentration of 0.5 g/L), the productivity increased by 6.4% compared to that when KOH was not added. At a concentration of KOH added of 1.1 g/L (potassium ion concentration of 0.8 g/L), the productivity increased by 9.3% compared to that when KOH was not added. At a concentration of KOH added of 1.5 g/L (potassium ion concentration of 1.0 g/L), the productivity increased by 8.3% compared to that when KOH was not added. It has been found that the productivity increases when the potassium ion concentration is 1.5 g/L compared to that when KOH is not added. However, it has been found that the productivity decreases when the potassium ion concentration is 1.8 g/L. Therefore, it has been found that O-acetyl-homoserine productivity is high when the potassium ion concentration is 0.3 g/L to 1.5 g/L.

From the above description, those skilled in the art to which the present disclosure belongs will be able to understand that the present disclosure can be implemented in other specific forms without changing the technical idea or essential features thereof. In this regard, it should be understood that the embodiments described above are exemplary in all respects and not restrictive. The scope of the present disclosure is defined by the appended claims rather than by the description preceding them, and therefore all changes or modifications derived from the meaning and scope of the claims and their equivalent concepts should be interpreted as being included in the scope of the present disclosure.

## Claims

1. A method for preparing O-acetyl-homoserine comprising a main culture step of culturing an O-acetyl-homoserine producing strain in a medium to produce O-acetyl-homoserine,
wherein a potassium salt is supplied to the medium.

2. The method for preparing O-acetyl-homoserine according to claim 1, wherein the potassium salt is at least one selected from the group consisting of potassium hydroxide (KOH), potassium acetate (KAc), potassium chloride (KCI), potassium sulfate (K₂SO₄), and potassium carbonate (K₂CO₃).

3. The method for preparing O-acetyl-homoserine according to claim 2, wherein the potassium salt is added so that a potassium ion concentration in the medium is 0.3 g/L to 1.5 g/L.

4. The method for preparing O-acetyl-homoserine according to claim 1, wherein a step of culturing the O-acetyl-homoserine producing strain in a flask and subsequently a seed culture step of culturing the O-acetyl-homoserine producing strain, before the main culture step is performed.

5. The method for preparing O-acetyl-homoserine according to claim 1, wherein a step of concentrating a fermented medium comprising O-acetyl-homoserine and a step of separating an O-acetyl-homoserine wet crystal is additionally performed after the main culture step is performed.

6. A medium comprising a carbon source, a nitrogen source, a phosphorus source, and a potassium salt,
wherein an O-acetyl-homoserine producing strain is capable to produce O-acetyl-homoserine cultured in the medium.

7. The medium according to claim 6, wherein the potassium salt is at least one selected from the group consisting of potassium hydroxide (KOH), potassium acetate (KAc), potassium chloride (KCI), potassium sulfate (K₂SO₄), and potassium carbonate (K₂CO₃).

8. The medium according to claim 7, wherein the potassium salt is added so that a potassium ion concentration in the medium is 0.3 g/L to 1.5 g/L.
